# EUROPEAN PATENT SPECIFICATION

(11) **EP 0 495 781 B1**
(45) Date of publication and mention of the grant of the patent: **27.08.1997**
(21) Application number: 90911203.9
(22) Date of filing: 18.06.1990
(51) Int. Cl.: A61K 51/02

(54) **RADIATION SYNOVECTOMY COMPOSITIONS**
RADIOSYNOVEKTOMIEZUSAMMENSETZUNGEN
COMPOSITIONS POUR LA RADIOSYNOVECTOMIE

(30) Priority: 12.10.1989 US 420795
(43) Date of publication of application: 29.07.1992
(73) Proprietor: MALLINCKRODT MEDICAL, INC., St. Louis, Missouri 63134 (US)
(72) Inventor: DEUTSCH, Edward, A., St. Louis, MO 63146 (US); LIBSON, Keren, F., St. Louis, MO 63146 (US); NOSCO, Dennis, L., Florissant, MO 63031 (US)
(74) Representative: Eyles, Christopher Thomas
(86) International application number: US9003392
(87) International publication number: WO9105570

(56) References cited:
- EP-A- 0 173 424
- US-A- 4 889 707
- Radiobiol.-Radiother., Volume 20, No. 3, 1979; H. DECKART et al.: "Radiosynovektomie des Kniegelenkes mit 198 Au-Kolloid, 90Y-Eisenhydroxyd-Kolloid und 186 Re-Sulfid-Kolloid1", pages 363-370
- International Journal of Applied Radiation and Isotopes, Volume 24, No. 1, 1973, Pergamon Press, GB; A. BARDY et al.: "Préparation de sulfure de thènium colloidal marqué par 186Re pour utilisation en synoviorthése", pp. 57-60
- The Journal of Bone and Joint Surgery, Inc., Volume 65-A, No. 3, March 1983; J. NOBLE et al.: "Leakage of Radioactive Particle Systems from a Synovial Joint Studied with a gamma Camera. Its Application to Radiation Synovectomy", pages 381-389
- La Nouvelle Presse Medicale, Volume 2, No. 20, 19 May 1973; F. DELBARRE et al.: "Une Nouvelle Preparation Radioactive pour la Synoviorthese: Le Rhenium 186 Colloidal. Advantages par Rapport au Colloide d'or 198", pages 1372-1373

## Description

The present invention relates to new radiation synovectomy compositions and to the use of such compositions. Certain of the compositions and their methods of preparation are also new.

Over two million people in the United States suffer from rheumatoid arthritis. The major cause of pain and physical disability for these individuals comes from destruction of the diarthroidal or synovial joints. The disease will involve the hands (metacarpophalangeal joints) for most of these patients, and over half will have affected knee joints. Untreated, the joint linings become increasingly inflamed resulting in pain, loss of motion and destruction of articular cartilage. One medical therapy applied to this disease involves the use of chemicals to attack and destroy the inflamed synovium (chemical synovectomy); however, the agents employed are highly toxic and capable of damaging articular cartilage. Similar concerns arise when repeated injections of corticoid steroids are used. In several cases where chemical therapy has failed, surgery is employed to remove the inflamed joint lining (surgical synovectomy). However, the difficulty of removing all the diseased synovium often leads to regrowth with recurrence of symptoms. If surgery is successful, freedom from symptoms usually last two to five years. When the symptoms reappear, surgical reintervention is not an option due to the presence of fibrosis and scar tissue which result from the previous surgery.

Radiation synovectomy has been used in Europe for many years to substantially oblate or destroy the inflamed synovium. The procedure is simple, involving only the injection of a radionuclide of the appropriate characteristics into the synovial cavity. The primary disadvantage of this technique has been the unacceptable radiation doses to non-target organ systems due to leakage of radioactive material from the cavity. The chemical nature of current radiation synovectomy agents is such that leaked material tends to be retained by the liver, spleen and lymph nodes. The leakage problem is often due either to the difficulty of formulating the correct particle size or lack of a tight binding of the nuclide to the particle. Another disadvantage is the use of radionuclides that don't have the appropriate beta energy to treat the inflamed synovium.

A radiation synovectomy agent that would not have the foregoing disadvantages would have the following characteristics:
1. The radionuclide used in the agent should have a beta energy sufficient to penetrate and oblate the enlarged synovial tissues, but not so great as to damage underlying articular cartilage or overlying skin. Any accompanying radiations should not generate an unacceptable extraneous radiation dose to the patient. The nuclide used may vary depending on the size of the joint and the beta energy necessary to oblate the synovial tissues in that joint.
2. The radionuclide should be attached to a particle of sufficient size so that it will not leak to any great extent from the diseased joint but still be able to be phagocytized in the synovium of the joint.
3. The binding between the radionuclide and the particle should be essentially irreversible through the course of radiotherapy (usually this duration of therapy is determined by the half life of the particular isotope).
4. The particle should preferably be biodegradable, i.e., it should be removable from the joint by the normal biological degradation mechanisms in the joint, itself, and should be cleared from the body in standard ways in a rapid manner with little or no toxicological effects.
5. If radioactive material should leak from the synovial cavity, the radionuclide should be released in a chemical form that rapidly egresses from the body, as for example, an anion which is excreted efficiently through the renal system. Preferably, the radionuclide would stay attached to a chelate or some portion of the degraded particle if this would facilitate clearance from the body.

An object of the present invention is to provide radiation synovectomy compositions containing a radiation synovectomy agent meeting substantially all of the foregoing criteria.

This invention relates to a radiation synovectomy composition for treating the inflamed synovium of a synovial joint of a person suffering from rheumatoid arthritis. It comprises a radionuclide complex bound to a substantially insoluble particle as the radiation synovectomy agent in a sufficient amount to provide satisfactory synovectomy when administered with a pharmaceutically acceptable radiation synovectomy vehicle. The radionuclide is a beta emitter that would substantially oblate or destroy the diseased synovium, but will not significantly damage underlying articular cartilages or overlying skin. The radionuclide complex is substantially kinetically stable but should degradation lead to leakage from the joint after administration, the radioactive material will rapidly clear from the body. The particle size of the agent is of sufficient size such that there is essentially little or no leakage of the intact radionuclide complex-particle unit from the synovial joint after administration. Additionally, the size and properties of the particle can be defined and controlled before it is bound to the radionuclide complex resulting in an agent having good synovectomy properties. Also, the binding of the radionuclide complex can be controlled resulting in better reproductivity and more complete binding.

A further feature of the present invention is the use of the radiation synovectomy compositions to treat inflamed synovia of people afflicted with rheumatoid arthritis. Another feature of the present invention is directed to novel radiation synovectomy agents.

As mentioned, the radiation agent comprises a substantially insoluble particle which is of suitable size as to not substantially leak from the joint after administration, that is to say from 1 to 10 microns, preferably from 2 to 5 microns. These particles are preferably biodegradable (but can also be degradable by other mechanisms) and not prone to aggregation under the conditions used to prepare or store the radiation synovectomy agent. The particle should have a density of approximately 0.7 to 1.3 gm/ml and should be suspendable in pharmaceutically acceptable vehicles. Some of the material from which such particles can be made include latex, derivatized polystyrene, silica, alumina, albumin (such as albumin microspheres), other proteins, polycarbonates, cellulose and inorganics, e.g., sulfur (colloid) or glass (beads). The particles have sites on the surface that permit covalent binding of the radionuclide complex. Such sites can include but are not limited to -NH₂, -SH, -OH, >C=O, and hydrophobic or hydrophilic regions or pockets. In addition to being insoluble, the particles must be non-toxic and preferably non*-*allergenic. Preferred particles include albumin microspheres and a sulfur colloid.

The radioisotopes that can be used are those that emit beta particles and are such that after administration will oblate the diseased synovium but will not significantly damage the underlying articular cartilage or overlying skin. These isotopes should have an average beta energy between 0.25 - 2.75 Mev, with or without an imageable gamma ray, with mean soft tissue penetration of about 0.70 and 25.0 mm, and with a half life of between 0.05 and 700 hours. Examples of preferred beta emitting isotopes include 198-Au, 188-Re, 186-Re, 177-Lu, 176m-Lu, 175-Yb, 169-Er, 166-Ho, 165-Dy, 156-Sm, 153-Sm, 115m-In, 105-Rh, 90-Y, 51-Cr, 77-As and 32-P. Preferably the isotope would either have an imageable gamma ray or could be doped with an isotope that would contain an imageable gamma ray. This doping isotope could be of the same or different element providing that its chemistry is sufficiently similar to the beta emitting isotope so that its biodistribution in the present use would be close or identical to the beta emitter. Preferred isotopes include: 186-Re, 188-Re, 90-Y, 153-Sm, 77-As and 105-Rh.

The radionuclide complexes that can be used are those that are stable before and after administration to the synovium joint. Additionally, if such complex leaks from the joint it will be rapidly cleared from the body. This will be the case even if the complex becomes separated from the insoluble particle. The complexes are formed by complexing the radionuclide under complexing conditions with a suitable ligand to provide a complex with the foregoing properties. Ligands that can be used are preferably polydentate, i.e., containing more than two coordinating atoms per ligand molecule. A coordinating atom is defined as one that has a free pair of electrons which can be bonded to the radionuclide. This atom is preferably separated by two or more atoms from any other coordinating atom. The coordinating atoms are chosen from nitrogen, oxygen, sulfur, phosphorus or carbon with nitrogen and/or oxygen and/or sulfur being the preferred coordinating atoms. Examples of chelates include MAG₃ (mercaptoacetylglycylglyclylglycine), all polycarboxylic acid-amine ligands especially DTPA (diethylenetriaminepentaacetic acid) e.g., EDTA (ethylenediaminetetraacetic acid), DADS and CO₂-DADS (N,N'-bis(mercaptoacetamido)ethylenediamine and N,N'-bis-(mercaptoacetamido)2,3-diaminopropanic acid) and their derivatives (European Application 0 173 424 and US Patent 4,673,562), mono- and poly-phosphonates, BATs (N,N'-bis(2-mercaptoethyl)-ethylenediamine) and derivatives (see European Applications 0 163 119, 0 200 211), thiosemicarbazones, PnAO and other amine-oxime ligands (European Applications 0 123 504 and 0 194 843), macrocyclic and open chain tetra-, penta-, hexa-, hepta-and octacoordinating nitrogen-containing compounds with and without other coordinating atoms or unsaturation.

Preferred ligands include MAG₃, DTPA, BAT, DADS and PnAO type ligands which have been modified so that they are bifunctional, i.e., can coordinate the radionuclide and also be coupled to the particle. Preferred complexes include: MAG₃ or DADS complexed with 186-Re, 188-Re, or 105-Rh.

The radiation agent of this invention can be prepared by attaching or binding to the particle the desired isotope under standard conditions for attachment. This involves coupling a ligand (either with or without a radioactive) to the particle with or without the presence of a spacer between the two units. Generally, the coupling can be done by any group(s) attached to the ligand that is (are) not crucial for complexing the radioisotope in a stable manner. This coupling portion of the ligand may consist of any group that can easily and specifically bind covalently to functional groups on the particle or that may simply adsorb very strongly to the surface of the particle. Examples of the covalent coupling would include activated esters of carboxylic acids which would combine covalently to amine groups, tosylates and acid halides which would combine with OH groups and maleimides which would combine with thiol groups, with the thiol, amine and OH groups assumed to be at or near the surface of the particle.

The following methods of preparing the desired radiation synovectomy agent may be used:
(a) The pre-formed method: One of the previously described radionuclide complexes is covalently bonded to one of the previously described particles having functional groups. Step one - a particle of the optimal size, (e.g. 1-10 microns) and composition (e.g. albumin, polycarbonate, cellulose, glass, latex) and having appropriate residues (amines, hydroxyls, carboxylates, thiols) is selected. Step two - a radioisotope (of the appropriate nuclear characteristics) which has been incorporated into a ligand (i.e., a radionuclide complex) is covalently bonded to the particle.
(b) The post-formed method: A ligand is covalently boned to one of the previously described particles. Thereafter, one of the previously described radioisotopes is incorporated into the covalently bonded complexing ligand, after the radionuclide has been treated in such a way, e.g., using a transfer ligand to facilitate transfer of the radionuclide to the ligand, to make it bind more readily to ligand.

Specific examples of methods (a) and (b) above are:
(a) The pre-formed method: A stabilizer (gentisic acid), a reductant (stannous) and a transfer agent (citrate) and the appropriate ligand are placed in a vial under an inert atmosphere. 188-Re or 186-Re as perrhenate is injected into the vial. This solution is heated for 15 to 30 minutes in a boiling water bath. The contents of the vial are removed with a syringe and injected into a second vial which contains the desired particle in an appropriate buffer solution. The contents of the second vial are treated in some fashion (heating, pH change) so as to effect covalent bonding of the metal chelate complex to the particle. Quality controls (tlc) are performed on the contents of this second vial. The labelled particles are suspended in a solution that is physically acceptable for injection.
(b) The post-formed method: Properly sized particles are slurried in a buffer solution with an excess of ligand that is activated in a fashion such that conjugation of the ligand to the particle is effected. This solution containing the resulting particle-ligand moiety is injected into a vial which contains a stabilizer, a transfer ligand and a reductant and into which perrhenate has been added in a previous step. The contents of this second vial are treated in some fashion (e.g., heating) so as to effect covalent attachment of the radiorhenium to the particle-bonded chelate. The labelled particles are suspended in a solution that is physically acceptable for injection. Preferred agents include:
   186-Re-MAG₃-albumin microspheres
   188-Re-MAG₃-albumin microspheres
   186-Re-MAG₃-sulfur colloid
   188-Re-MAG₃-sulfur colloid
   186-Re-DADS-albumin microspheres
   188-Re-DADS-albumin microspheres
   186-Re-DADS-sulfur colloid
   188-Re-DADS-sulfur colloid

The radiation synovectomy agents of this invention may be used in any pharmaceutically acceptable radiation synovectomy vehicle. These include those suitable for injection, such as aqueous buffer solutions, e.g. (trishydroxymethyl)aminomethane and its salts, phosphate, citrate, bicarbonate, e.g., sterile water for injection, physiological saline and balanced ionic solutions containing chloride and/or bicarbonate salts of normal blood plasma cations such as calcium, sodium, potassium, magnesium. Other buffer solutions are described in Remington's Practice of Pharmacy, 11th Edition, for example on page 170. Additionally, the vehicle may contain stabilizers, antioxidants and other adjuncts. Stabilizers include gelatin or other materials in stabilizing amounts to prevent aggregation of the particles, antioxidants in antioxidant amounts such as reducing sugars (e.g. fructose, or free acid or metal salts of gentisic acid) ascorbic acid and other adjuvants such as reducing agents, preferably stannous salts, intermediate exchange ligands in exchange amounts such as metal salts of tartrate, gluconate or citrate as well as bulking agents in bulking amounts such as lactose.

The composition may be formulated in a one-step procedure as a lyophilized kit where the radioisotope solution is injected for reconstitution or as an autoclaved or radiation sterilized solution which is then treated with the radioisotope. In this case, the ligand has already been attached to the particle before lyophilization or autoclaving. The product may be formulated in a two-step scheme where the radioisotope is bound to the ligand and then this complex with or without purification as necessary is combined with the particles to give the final radiation synovectomy composition. Any of these steps may require heating and any of the intermediates or final products may require purification before use.

The concentration of the radiation synovectomy agent in the pharmaceutically acceptable vehicle varies with the particular use. A sufficient amount is present to provide satisfactory radiation synovectomy. This amount will vary with the physical properties of the isotope being used. For example, when using 186-Re for radiation synovectomy of the hip, the concentration is sufficient to provide 2 to 5mCi and preferably from 3 to 4mCi. When it is used for the radiation synovectomy of the wrist joints, it is used in an amount from 1 to 3mCi and preferably from 1 to 2mCi.

The radiation synovectomy composition is administered so that preferably it remains substantially in the joint for 20 half-lifes of the isotope although shorter residence times are acceptable as long as the leakage of the radionuclide is small and the leaked radionuclide is rapidly cleared from the body.

The radiation synovectomy compositions may be used in the usual way for such procedures. For example, in the case of the treatment of a knee-joint, a sufficient amount of the radiation synovectomy composition to provide adequate radiation synovectomy is injected into the knee-joint. There are a number of different techniques which can be used and the appropriate technique varies on the joint being treated. An example for the knee joint has been excerpted below from Nuclear Medicine Therapy, J. C. Harbert, J.S. Robertson and K.D. Reid, 1987, Thieme Medical Publishers, pages 172-3.

Strict asepsis is essential. The area to be aspirated and/or injected should be cleansed and prepped as for a spinal tap.

The injection site is selected by first obtaining radiographs in two planes with the joint position at the injection angle. These are used to correlate easily palpable bony landmarks as a guide for needle placement. Major nerves, vessels and tendons should be avoided. Extensor surfaces are the preferred injection sites. The specific area of the joint to be injected is then marked with firm pressure by a ballpoint pen which has the writing tip retracted. This will leave an impression lasting 10 to 30 minutes. The area is carefully cleansed with Betadine solution and the injection site is anesthetized with 1% xylocaine. The injection needle is then inserted through the ballpoint impression, using care to avoid hitting the cartilage. Following insertion, the needle position is checked fluoroscopically using a few milliliters of contrast material. Alternatively 1 mCi (37MBq) of ^{99m}Tc-sulfur colloid can be injected prior to injecting the therapeutic dose. The joint is then scanned to assure distribution throughout the joint space. This is an important precaution, because loculated distribution is probably a common cause of treatment failure. Following injection of radiocolloid the needle is flushed with 10 to 20 mg triamcinolone and the needle withdrawn. The joint is then splinted or the patient confined to bed rest for 48 hours to minimize leakage from the joint space (in the case of ¹⁶⁵Dy-macroaggregates, 7 hours bed rest is deemed sufficient.)

The knee is the easiest joint to inject. The patient should be in a supine position with the knee fully extended. The puncture is made 1 to 2 cm medial to the medial margin of the patella using an 18-gauge by 1.5 in. needle directed slightly inferiorly and toward the joint space. The joint space should be entered and easily aspirated. If osteophytes make this approach difficult, the knee may be injected with the patient sitting and the knee fixed. In this case the needle is placed beneath the distal border of the patella and directed straight posteriorly or slightly superiorly toward the joint cavity.

In most cases after the joint has been injected, it is either (1) moved to allow homogeneous distribution of the radiation synovectomy agent and then immobilized and shielded with appropriate radioactive shielding for a period of time related to the half-life of the isotope or (2) simply immobilized and shielded without working the joint.

It is to be understood that the invention is not to be limited to the exact details of operation or exact compounds, compositions or procedures shown and described, as obvious modifications and equivalents will be apparent to one skilled in the art, and the invention is therefore to be limited only by the scope of the appended claims.

## Claims

1. A radiation synovectomy composition for treating inflamed synovium containing a radionuclide complex bound to a substantially insoluble particle having a size in the range of from 1 to 10 microns as the radiolabelled synovectomy agent in a sufficient amount to provide satisfactory synovectomy of the inflamed synovium of a joint together with a pharmaceutically acceptable vehicle, said radionuclide being a beta emitter that will substantially oblate the inflamed synovium, but not significantly damage underlying articular cartilage, said radionuclide complex being substantially kinetically stable, and if said radionuclide or radionuclide complex leaks from said joint, being rapidly excretable from the body.

2. A composition according to claim 1, wherein said particle is selected from albumin microspheres, sulfur colloid, glass beads, albumin, and latex.

3. A composition according to claim 1 or claim 2, wherein said complex is selected from a 188-Re complex and a 186-Re complex.

4. A composition according to claim 3, wherein said complex is selected from 188-Re MAG₃ complex, 186-Re MAG₃ complex, 186-Re DADS complex and 188-Re DADS complex.

5. A composition according to claim 4, wherein said particle is albumin and said complex is 188-Re MAG₃ or 186-Re MAG₃ bound covalently to albumin through the pendant carboxylate group.

## Patentansprüche

1. Radiosynovektomie-Zusammensetzung zur Behandlung der entzündeten Synovialis, die zusammen mit einem pharmazeutisch zulässigen Träger als radioaktiv markiertes Synovektomiemittel einen Radionuklid-Komplex in Bindung an ein im wesentlichen unlösliches Teilchen einer Größe in dem Bereich von 1 bis 10 Mikron in einer zur Schaffung einer genügenden Synovektomie der entzündeten Synovialis eines Gelenks ausreichenden Menge enthält, wobei das Radionuklid ein Betastrahler ist, der die entzündete Synovialis im wesentlichen beseitigt, jedoch das darunter liegende Gelenkknorpelgewebe nicht signifikant schädigt, und der Radionuklid-Komplex im wesentlichen kinetisch stabil ist und schnell aus dem Körper ausscheidbar ist, wenn das Radionuklid oder der Radionuklid-Komplex das Gelenk verlässt.

2. Zusammensetzung nach Anspruch 1, bei der das Teilchen unter Albumin-Mikrokugeln, Schwefelkolloid, Glasperlen, Albumin und Latex ausgewählt ist.

3. Zusammensetzung nach Anspruch 1 oder Anspruch 2, bei der der Komplex aus einem 188-Re-Komplex und einem 186*-*Re-Komplex ausgewählt ist.

4. Zusammensetzung nach Anspruch 3, bei der der Komplex aus 188-Re-MAG₃-Komplex, 186-Re-MAG₃-Komplex, 186-Re-DADS-Komplex und 188-Re-DADS-Komplex ausgewählt ist.

5. Zusammensetzung nach Anspruch 4, bei der das Teilchen Albumin ist und der Komplex 188*-*Re-MAG₃ oder 186*-*Re-MAG₃ ist, der durch die anhängende Carboxylatgruppe kovalent an Albumin gebunden ist.

## Revendications

1. Composition de radiosynovectomie destinée au traitement d'une membrane synoviale enflammée, contenant un complexe de radionucléide lié à une particule sensiblement insoluble, ayant une dimension comprise dans l'intervalle de 1 à 10 µm, en tant qu'agent de synovectomie radiomarqué, en une quantité suffisante pour assurer une synovectomie satisfaisante de la membrane synoviale enflammée d'une articulation, ainsi qu'un véhicule pharmaceutiquement acceptable, ledit radionucléide étant un émetteur de rayons β qui raccourcira sensiblement la membrane synoviale enflammée, mais sans endommager significativement le cartilage articulaire sous-jacent, ledit complexe de radionucléide étant sensiblement cinétiquement stable et étant rapidement excrétable hors de l'organisme si ledit radionucléide ou complexe de radionucléide fuit hors de ladite articulation.

2. Composition selon la revendication 1, où ladite particule est sélectionnée parmi des microsphères d'albumine, un colloïde soufré, des billes de verre, l'albumine et le latex.

3. Composition selon la revendication 1 ou la revendication 2, où ledit complexe est sélectionné parmi un complexe de Re-188 et un complexe de Re-186.

4. Composition selon la revendication 3, où ledit complexe est sélectionné parmi un complexe de MAG₃-Re-188, un complexe de MAG₃-Re-186, un complexe de DADS-Re-186 et un complexe de DADS-Re-188.

5. Composition selon la revendication 4, où ladite particule est de l'albumine et ledit complexe est MAG₃-Re-188 ou MAG₃-Re-186 lié par covalence à de l'albumine par le groupe carboxylate latéral.
